(19) **Europäisches Patentamt / European Patent Office / Office européen des brevets**

(11) **EP 4 682 532 A1**

(12) # EUROPEAN PATENT APPLICATION

(43) Date of publication:
21.01.2026 Bulletin 2026/04

(21) Application number: 24189662.0

(22) Date of filing: 19.07.2024

(51) International Patent Classification (IPC):
*G01N 33/00* (2006.01)    *G16C 60/00* (2019.01)

(52) Cooperative Patent Classification (CPC):
**G01N 33/0001; G16C 60/00;** G16C 20/20;
G16C 20/70

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: **Nicoventures Trading Limited**
London WC2R 3LA (GB)

(72) Inventors:
- **FLORES, DOUGLAS WILLIAM MENEZES**
  20.031-170 Rio de Janeiro (BR)
- **KAISER, SAMUEL**
  20.031-170 Rio de Janeiro (BR)
- **CANOVA, LUCIANA**
  20.031-170 Rio de Janeiro (BR)
- **ASSIS, CAMILA**
  20.031-170 Rio de Janeiro (BR)

- **NESPECA, MAURÍLIO**
  20.031-170 Rio de Janeiro (BR)
- **DOS SANTOS DE SOUZA, VINICIUS WELLINGTON**
  20.031-170 Rio de Janeiro (BR)
- **DE SOUZA NETTO, JOSÉ MARCELINO**
  20.031-170 Rio de Janeiro (BR)
- **DE AZEVEDO, JULIA LILIAN GAUDERTH**
  20.031-170 Rio de Janeiro (BR)
- **LIMA, PAMELA**
  20.031-170 Rio de Janeiro (BR)
- **MEDIANEIRA FAVERO PORTE, LILIANE**
  20.031-170 Rio de Janeiro (BR)
- **MARCELO, MARCELO CAETANO ALEXANDRE**
  London, WC2R 3LA (GB)
- **WRIGHT, BRADLEY GUY**
  London, WC2R 3LA (GB)

(74) Representative: **Gill, Siân Victoria**
**Venner Shipley LLP**
**200 Aldersgate**
**London EC1A 4HD (GB)**

(54) **MACHINE LEARNING FOR DETERMINING SENSORY PROPERTIES OF PROCESSED TOBACCO PRODUCT**

(57)    The invention relates to computer implemented methods for determining sensory attributes of a processed tobacco product.

FIG. 1

## Description

## Technical Field

Methods and systems for determining properties, components and/or sensory attributes of processed tobacco product samples are described herein.

## Background

Sensory evaluation plays an important role in the assessment of the quality of a tobacco product. The determination of tobacco sensory attributes can be used for product quality control, crop consistency ensuring, blend designs, estimation of product shelf-life, among other applications. Human sensory analysis is the main method that is currently used for evaluating tobacco product quality and consumer acceptance.

The consumer experience of tobacco typically occurs through the use of a tobacco industry product, and is characterised by various sensory inputs relating to flavour, taste, aroma, etc. Various attempts have been made break down a given flavour or taste into a number of attributes, such as bitterness, dryness, etc. The attributes then form a multi-dimensional measurement system for assessing tobacco taste from a consumer perspective. However, because such attributes are semi-qualitative in nature, they are generally assessed by people, such as a human sensory panel, using the tobacco industry product, which makes reproducibility more difficult.

Manufacturers of tobacco industry products typically want to provide consumers with a consistent and reliable product, including in terms of the various sensory attributes mentioned above. It can be challenging to achieve such consistency, given that tobacco is a natural product. Thus, the tobacco is subject to intrinsic variation between individual plants, combined with additional variations caused by differences in growing location, soil, agricultural management, etc. Indeed, even tobacco grown at a single location may experience fluctuations in properties, for example, based on changes in climate (e.g. whether the growing period has been relatively hot and dry or cold and wet) and/or details of subsequent processing for the tobacco (such as curing). These difficulties may be compounded by having multiple different varieties in a tobacco blend, although on the other hand, a blend is often performed to try to compensate for such variation.

## Summary

According to a first aspect of this specification, there is described a computer implemented method for determining sensory attributes of a processed tobacco product. The method comprises: obtaining, by one or more processors, mass spectroscopy data from a processed tobacco product; determining, by the one or more processors, one or more chemical fingerprints of the processed tobacco product from the mass spectroscopy data; and processing, by the one or more processors, the one or more chemical fingerprints of the processed tobacco product using a plurality of machine learning models to obtain a respective score for each of a plurality of sensory attributes for the processed tobacco product.

The plurality of machine learning models may comprise: a first plurality of machine learning models configured to take one or more chemical fingerprints of a tobacco blend of the processed tobacco product as input, and output respective scores for the plurality of sensory attributes; and a second plurality of machine learning models configured to take one or more chemical fingerprint of an emission of the processed tobacco product as input, and output respective scores for the plurality of sensory attributes.

The processed tobacco product may comprise a tobacco blend. The processed tobacco product may comprise a tobacco heating product sample.

The plurality of sensory attributes may comprise a plurality of pre-heating sensory attributes for the processed tobacco product. The plurality of pre-heating sensory attributes for the processed tobacco product may comprise one or more of: a rod aroma intensity; and/or an ease of rod insertion.

The plurality of sensory attributes may comprise a plurality of first-puff sensory attributes for the processed tobacco product, wherein the first puff attributes relate to initial sensory attributes of the processed tobacco product when heated. The plurality of first-puff sensory attributes may comprise: an impact attribute; an irritation attribute; a visible emission attribute; a taste intensity attribute; a flavour intensity attribute; an emission volume attribute; and/or an emission warmth attribute.

The plurality of sensory attributes may comprise a plurality of overall sensory attributes for the processed tobacco product, wherein the plurality of overall sensory attributes for the processed tobacco product relate to sensory attributes of the processed tobacco product during heating. The plurality of overall sensory attributes may comprise one or more of: cardboard-paper intensity; a bread-cereal intensity; an emission volume; a tobacco taste intensity; an off notes intensity; an impact attribute; an irritation attribute; a visible emission attribute; a roasted intensity attribute; a spice intensity attribute; a woody intensity attribute; a bitterness attribute; a sweetness attribute; a sourness attribute; a flavour amplitude; an emission warmth; a draw resistance a flavour consistency; and/or an emission consistency.

The plurality of sensory attributes may comprise a plurality of aftertaste sensory attributes for the processed tobacco product, wherein the plurality of aftertaste sensory attributes for the processed tobacco product relate to an aftertaste of the tobacco product. The plurality of aftertaste sensory attributes for the processed tobacco product may comprise one or more of: a flavour intensity; a mouth drying attribute; an oily mouth coating attribute; a tobacco taste intensity; an ease of rod removal attribute; an aroma on hand attribute; and/or a bitter taste intensity.

The plurality of machine learning models may comprise a plurality of regression methods, such as Partial Least Square regression, combined with variable selection methods, such as Variable Importance in Projection method.

The mass spectroscopy data may be obtained from a sample from a manufactured batch of the processed tobacco product. The method may further comprise: determining that the scores for one or more of the plurality of sensory attributes for the processed tobacco product are not within a respective one or more threshold values of corresponding reference scores for the processed tobacco product; and in response to determining that the scores for one or more of the plurality of sensory attributes for the processed tobacco product are not within the respective one or more threshold values of the corresponding reference scores for the processed tobacco product, indicating that the manufactured batch of the processed tobacco product is rejected.

The method may further comprise: comparing the respective scores for each of the plurality of sensory attributes to corresponding ground-truth sensory data for the processed tobacco product using one or more objective functions; and updating parameters of the plurality of machine-learning models based on the comparison. The ground-truth sensory data may comprise signature profiling data obtained from a plurality of human testers.

According to a further aspect of this specification, there is described apparatus comprising one or more processors and a memory, the memory storing computer readable instructions that, when executed by the one or more processors, cause the apparatus to perform a method according to any one or more of the methods described herein.

According to a further aspect of this specification, there is described a computer program product storing computer readable instructions that, when executed by a computer, cause the computer to perform a method according to any one or more of methods described herein.

In any of the embodiments described herein, the method may be implemented using a numerical computing environment and/or framework.

In any of the embodiments described herein, the numerical computing environment and/or framework may comprise using any IDE (Integrated Development Environment). Examples of these may comprise PyCharm, Spyder, Jupiter, R Studio; or any Numerical-software packages, or any of MATLAB, GNU Octave, PyCharm, and R Studio. The embodiments described herein are not limited to these examples, however.

In any of the embodiments described herein, the method may be implemented using one or more programming languages.

In any of the embodiments described herein, said one more programming languages may comprise any general purpose programming language, for example any one or more of Python, C, C++, C#, R, Julia and Java. The embodiments described herein are not limited to these examples, however.

**Brief Description of the Drawings**

Embodiments of the present invention will now be described, by way of example only, with reference to the accompanying drawings.

Figure 1 depicts an overview of an example method for determining sensory attributes of a processed tobacco product;

Figure 2A depicts an overview of an example method for training a machine-learning model to determine sensory attributes of a processed tobacco product;

Figure 2B depicts an overview of a further example method for training a machine-learning model to determine sensory attributes of a processed tobacco product;

Figure 3 shows a flow diagram of an example method for determining sensory attributes of a processed tobacco product;

Figure 4 shows a flow diagram of an example method for training a machine-learning model to determine sensory attributes of a processed tobacco product; and

Figure 5 shows a schematic overview of an example computing system.

**Detailed Description**

The systems and methods described herein provide a means by which sensory attributes of a processed tobacco product can be predicted using machine learning methods. The predicted sensory attributes can be used as a means of quality control for batches of processed tobacco products. Alternatively or additionally, the predicted sensory attributes can be used as a means of analysing a new/potential processed tobacco product to predict whether it has desirable sensory properties for an end user.

The systems and methods described herein can result in more reproducible sensory analysis of processed tobacco products when compared to human testing, which can in turn lead to improved consistency in quality control methods.

As used herein, the term "processed tobacco product" refers to a tobacco industry product comprising processed tobacco. Such products may be or may form part of a delivery system.

As used herein, the term "delivery system" is intended to encompass systems that deliver at least one substance to a user, and includes:

combustible aerosol provision systems, such as cigarettes, cigarillos, cigars, and tobacco for pipes or for roll-your-own or for make-your-own cigarettes (whether based on tobacco, tobacco derivatives, expanded tobacco, reconstituted tobacco, tobacco substitutes or other smokable material);

non-combustible aerosol provision systems that release compounds from an aerosol-generating material without combusting the aerosol-generating material, such as electronic cigarettes, tobacco heating products, and hybrid systems to generate aerosol using a combination of aerosol-generating materials; and

aerosol-free delivery systems that deliver the at least one substance to a user orally, nasally, transdermally or in another way without forming an aerosol, including but not limited to, lozenges, gums, patches, articles comprising inhalable powders, and oral products such as oral tobacco which includes snus or moist snuff, wherein the at least one substance may or may not comprise nicotine.

According to the present disclosure, a "combustible" aerosol provision system is one where a constituent aerosol-generating material of the aerosol provision system (or component thereof) is combusted or burned during use in order to facilitate delivery of at least one substance to a user.

In some embodiments, the processed tobacco product is selected from the group consisting of a cigarette, a cigarillo and a cigar.

According to the present disclosure, a "non-combustible" aerosol provision system is one where a constituent aerosol-generating material of the aerosol provision system (or component thereof) is not combusted or burned in order to facilitate delivery of at least one substance to a user.

In some embodiments, the delivery system is a non-combustible aerosol provision system, such as a powered non-combustible aerosol provision system.

In some embodiments, the non-combustible aerosol provision system is an electronic cigarette, also known as a vaping device or electronic nicotine delivery system (END), although it is noted that the presence of nicotine in the aerosol-generating material is not a requirement.

In some embodiments, the non-combustible aerosol provision system is an aerosol-generating material heating system, also known as a heat-not-burn system. An example of such a system is a tobacco heating system.

In some embodiments, the processed tobacco product is a consumable for use in a tobacco heating system.

In some embodiments, the non-combustible aerosol provision system is a hybrid system to generate aerosol using a combination of aerosol-generating materials, one or a plurality of which may be heated. Each of the aerosol-generating materials may be, for example, in the form of a solid, liquid or gel and may or may not contain nicotine. In some embodiments, the hybrid system comprises a liquid or gel aerosol-generating material and a solid aerosol-generating material. The solid aerosol-generating material may comprise, for example, tobacco or a non-tobacco product.

In some embodiments, the processed tobacco product is a consumable for use in a hybrid system.

In some embodiments, the disclosure relates to consumables comprising aerosol-generating material and configured to be used with non-combustible aerosol provision devices. These consumables are sometimes referred to as articles throughout the disclosure.

A consumable is an article comprising or consisting of aerosol-generating material, part or all of which is intended to be consumed during use by a user. A consumable may comprise one or more other components, such as an aerosol-generating material storage area, an aerosol-generating material transfer component, an aerosol generation area, a housing, a wrapper, a mouthpiece, a filter and/or an aerosol-modifying agent. A consumable may also comprise an aerosol generator, such as a heater, that emits heat to cause the aerosol-generating material to generate aerosol in use. The heater may, for example, comprise combustible material, a material heatable by electrical conduction, or a susceptor.

In some embodiments, the non-combustible aerosol provision system, such as a non-combustible aerosol provision device thereof, may comprise a power source and a controller. The power source may, for example, be an electric power source or an exothermic power source. In some embodiments, the exothermic power source comprises a carbon substrate which may be energised so as to distribute power in the form of heat to an aerosol-generating material or to a heat transfer material in proximity to the exothermic power source.

In some embodiments, the non-combustible aerosol provision system may comprise an area for receiving the consumable, an aerosol generator, an aerosol generation area, a housing, a mouthpiece, a filter and/or an aerosol-modifying agent.

In some embodiments, the consumable for use with the non-combustible aerosol provision device may comprise aerosol-generating material, an aerosol-generating material storage area, an aerosol-generating material transfer component, an aerosol generator, an aerosol generation area, a housing, a wrapper, a filter, a mouthpiece, and/or an aerosol-modifying agent.

In some embodiments, the delivery system is an aerosol-free delivery system that delivers at least one substance to a user orally, nasally, transdermally or in another way without forming an aerosol, including but not limited to, lozenges, gums, patches, articles comprising inhalable powders, and oral products such as oral tobacco which includes snus or moist snuff, wherein the at least one substance may or may not comprise nicotine.

In some embodiments, the processed tobacco product comprises a substance to be delivered. The product may comprise one or more active constituents, one or more flavours, one or more aerosol-former materials, and/or one or more other functional materials. The one or more other functional materials may comprise one or more of pH regulators, colouring agents, preservatives, binders, fillers, stabilizers, and/or antioxidants.

The methods disclosed herein may be used to identify or predict the sensory attributes of the tobacco composition or blend used in the processed tobacco product. Additionally or alternatively, the methods may be used to identify or predict the sensory attributes of the processed tobacco product upon use. For example, the methods may be used to determine the sensory attributes associated with the use of a particular tobacco heating device or heating according to a particular temperature profile. Also, the sensory impact of particular design features of a consumable may also be determined, such as pressure drop or ventilation, or different filters or mouth end elements.

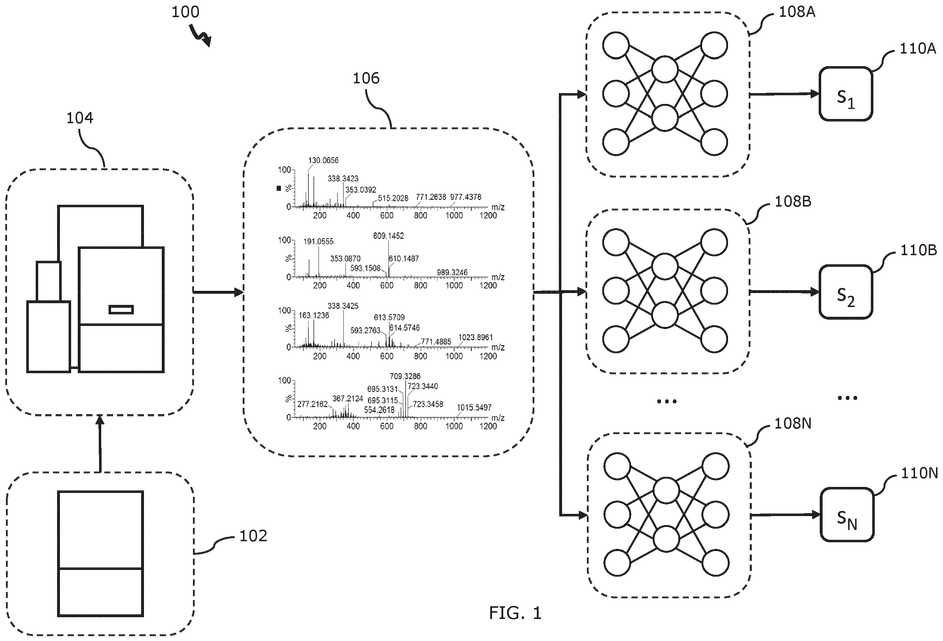

Figure 1 depicts an overview of an example method 100 for determining sensory attributes of a processed tobacco product.

A sample 102 of a processed tobacco product is obtained. An analysis 104, e.g., mass spectroscopy, is performed on the sample 102 to obtain a chemical fingerprint 106 of the sample 102. The chemical fingerprint 106 is processed by a plurality of machine learning (ML) models 108A-N, which generate a score 110A-N of each of a plurality of sensory attributes for the sample 102.

The sample 102 of the processed tobacco product may be extracted using a predefined method that corresponds to the method used to generate training data for the plurality of machine learning models 108A-N. Any method known in the art for extracting samples 102 may be used.

The chemical fingerprint 106 of the processed tobacco product can be generated using a mass spectroscopy process. For example, high-throughput screening by flow injection coupled to high-resolution mass spectrometry (HTS-FIA-HRMS) can be performed to generate the chemical fingerprint 106. The chemical fingerprint may comprise, for example, $O(10^5)$ chemical features per sample, e.g., 20,000.

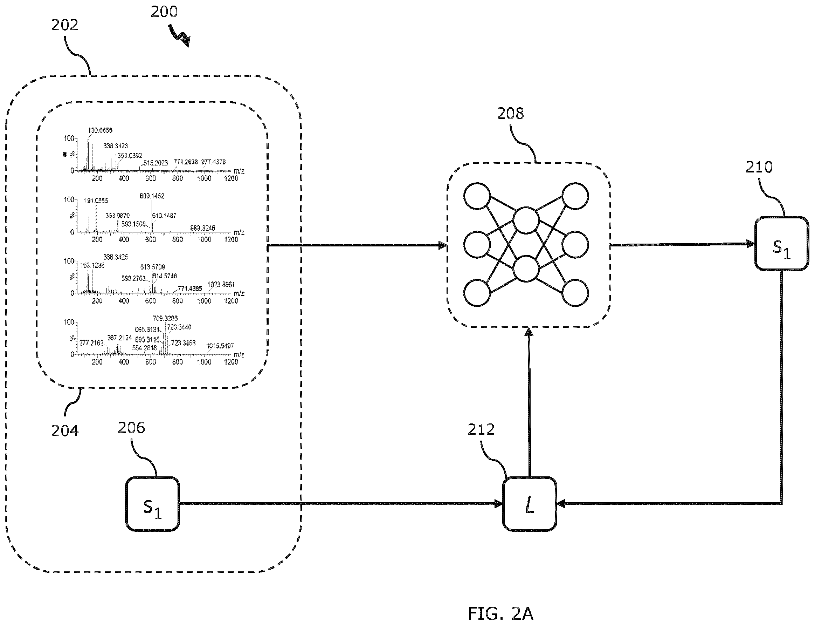

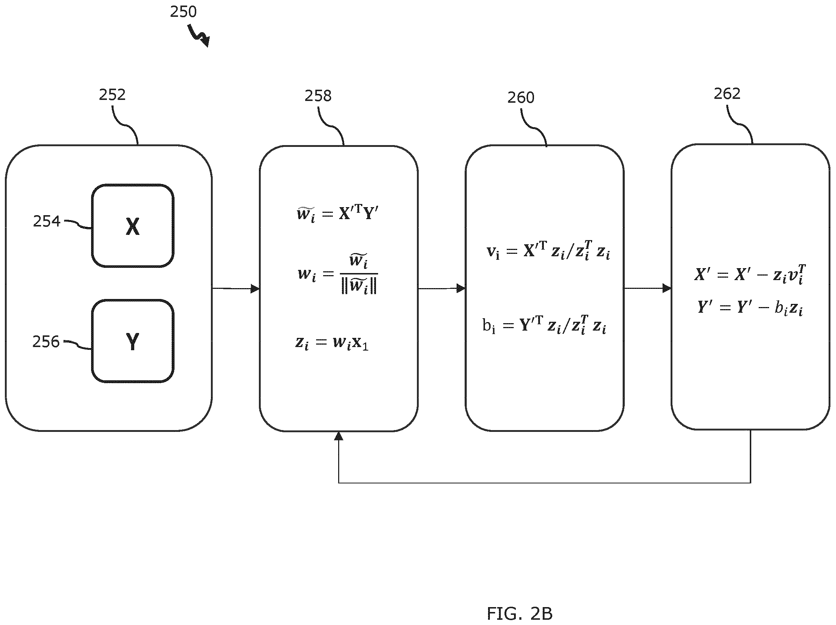

The plurality of machine learning models 108A-N are each specialised for predicting a particular one or more sensory attributes. For example, each machine learning model may be associated with one sensory attribute. The machine learning models 108A-N have each been trained to predict their respective scores using a machine learning method, such as the method described in relation to figures 2A and/or 2B.

Each machine learning model 108A-N receives at least a respective portion of the features from the chemical fingerprint 106, and processes the features based on values of (learned) parameters of the machine learning model to generate a predicted score 110A-N for a sensory attribute.

The machine learning models in the plurality of machine learning models may be any suitable parametrised model known in the art. For example, the plurality of machine learning models may comprise one or more (e.g., a plurality) of partial least square regression models. Alternatively or additionally, the plurality of machine learning models may comprise one or more (e.g., a plurality) of regression models which can be developed by one or more regression methods, such as support vector regression, random forest regression, k-nearest neighbour regression, artificial neural network regression methods, e.g., fully connected neural networks, convolutional neural networks, residual networks, and/or the like, but not limited to the mentioned methods. In addition, the machine learning models can be combined or not with variable selection methods, such as Variable Importance in Projection, Genetic Algorithm, Interval Partial Least Square, Stepwise, Selectivity Ration, Successive Projection Algorithm, and others.

Each score 110A-N may be a numerical score in a respective predetermined range for the corresponding sensory attribute, e.g., between zero and five, or between zero and ten. The set of potential scores 110A-N for a sensory attribute may, in some examples, take substantially continuous values in the predetermined range. Alternatively, the set of potential scores 110A-N for a sensory attribute may take discrete values in the predetermined range, e.g., only integer values in the range.

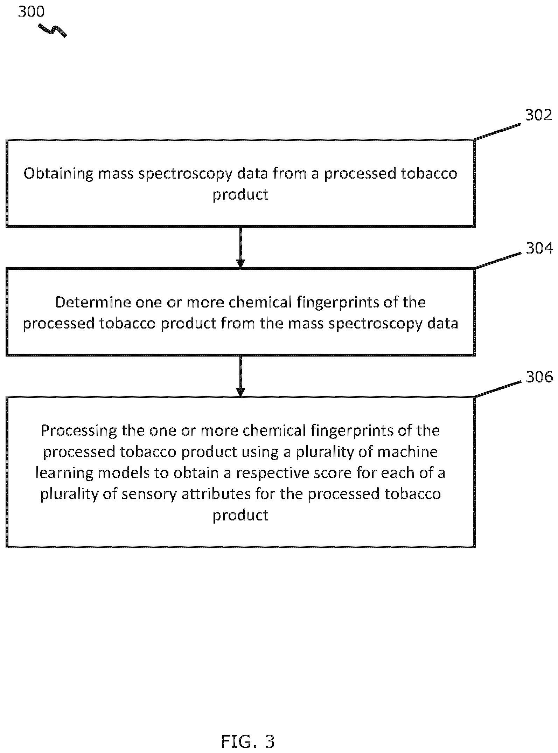

Each score 110A-N represents a sensory attribute associated with the processed tobacco product. In some examples, scores 110A-N may be grouped into subsets of scores based on which phase of use of the processed tobacco product they relate to. For example, a plurality of scores relating to "before heating" sensory aspects may be grouped together, a plurality of scores relating to "first puff" sensory aspects may be grouped together, a plurality of scores relating to "during/overall" sensory aspects may be grouped together, and/or a plurality of scores relating to "after taste" sensory aspects may be grouped together. Examples of such sensory attributes are described herein in relation to figure 3.

The method 100 may be used as part of a quality control method. In such examples, the sample 102 of the processed tobacco product is taken from a batch of manufactured tobacco products. The processed tobacco product being manufactured is associated with a reference set of sensory attribute scores, e.g., individual scores or ranges of scores. The sample 102 is processed as described in relation to figure 1 to generate a plurality of sensory attribute scores 110A-N for the sample 102. These sensory attribute scores 110A-N are compared to respective reference sensory attribute scores and, based on the comparison, it is determined whether the batch passes the quality control test or not.

For example, each score in the reference set of sensory attribute scores can be associated with one or more respective threshold values, e.g., an upper threshold value and a lower threshold value, or a single threshold value. The refence score and the one or more threshold values together define a range of "acceptable" values for a sensory attribute.

If each sensory attribute score 110A-N is within its respective range of reference values, then the batch from which the sample 102 was taken passes the quality control test. If one or more of the sensory attribute scores 110A-N is outside of the respective range of reference values, then the batch from which the sample 102 was taken fails the quality control test.

Figure 2A depicts an overview of an example method 200 for training a machine-learning model to determine one or more sensory attributes of a processed tobacco product.

A training example 202 is obtained from a training dataset (not shown). The training example 202 comprises a chemical fingerprint 204 of a processed tobacco product and one or more ground truth sensory attribute scores 206 for the processed tobacco product. A set of features from the chemical fingerprint 204 is input into a machine learning model 208, which generates one or more candidate sensory attribute scores 210 from the set of features of the chemical fingerprint 204. The one or more candidate sensory attribute scores 210 are compared to corresponding ground truth sensory attribute scores 206 of the training example 202 using, for example, an objective/loss function 212. Based on the comparison, parameters of the machine learning model 208 are updated. The method 200 may be iterated to train the machine learning model 208 to predict a sensory attribute score from a chemical fingerprint.

The training dataset comprises a plurality of training examples, each training example comprising a chemical fingerprint of a respective processed tobacco product and one or more (e.g., a plurality of) ground truth sensory attribute scores for that processed tobacco product. Each training example may relate to different processed tobacco product. The training dataset may comprise at least 50 training examples, for example at least 100 training examples or at least 150 raining examples.

The ground truth sensory attribute scores for a processed tobacco product can be generated based on human testing/evaluation. A panel of human testers may use the processed tobacco product, and each provide a score of each of a plurality of sensory attributes for the processed tobacco product, e.g., a numerical score in a predefined range for each sensory attribute. The ground truth sensory attribute score for a particular attribute can be generated by averaging the scores for that attribute provided by the panel of human testers, e.g., by taking the mean, median or modal score for the attribute.

The machine learning model(s) 208 process the chemical fingerprint 204 of the training example 202 as described in relation to figure 1 to generate the candidate sensory attribute score 210.

The loss/objective function 212 is based on a difference between the candidate sensory attribute score 210 and the corresponding ground truth sensory attribute score 206. The objective function may, in some examples, be based on an average (e.g., a mean) of differences between a plurality of candidate sensory attribute score 210 and the corresponding ground truth sensory attribute score 206 in a batch of training examples 202. Examples of loss/objective function include, but are not limited to, an L2 or L1 loss, and/or a classification loss (such as a cross entropy loss).

To generate the updates for the parameters of the machine learning model 208, an optimisation routine may be applied to the objective/loss function 212, with the goal of maximising or minimising the objective function 212 (depending on the form of objective function used). Examples of such optimisation routines include, but are not limited to, gradient descent methods, such as stochastic gradient descent.

The parameters of the machine learning model 208 updated base on the objective/loss function 212 depend on the type of machine learning model 208 being trained. For example, for an artificial neural network model, the updated parameters may comprise weights and/or biases of the neural network.

Figure 2B depicts a further example method 250 for training a machine-learning model to determine one or more sensory attributes of a processed tobacco product. The method 250 is an example of a partial least squares method that can be used to train a partial least squares model on a training dataset 252. The goal of the method is to determine a linear model that links features of the chemical fingerprints 254 of tobacco products (collectively denoted $\mathbf{X}$) to their corresponding sensory attribute scores (collectively denoted $\mathbf{Y}$) 256 for a particular sensory attribute. Partial least square methods can be beneficial as the number of features in the chemical fingerprint 254 (e.g. $O(10^5)$) is much higher than the number of samples or scores ($O(1)$). The example described here is one example of a partial least squares regression method; other regression methods may alternatively be used.

The method 250 uses a training dataset 252 comprising a set of $n$ chemical fingerprints 254, $\mathbf{X}$, of a processed tobacco product and a set of $n$ corresponding sets of one or more sensory attribute scores 256, $\mathbf{Y}$. The training dataset 252 may be obtained using any of the methods described in relation to figure 2A. The set of chemical fingerprints 254 may be represented as an $n \times p$ dimensional matrix, where $p$ is the number of fingerprint features. The set of sensory attributes scores 256 may be represented as an $n$-dimensional vector (for a single sensory attribute score per chemical fingerprint) or a $n \times l$ dimensional matrix (for $l$ sensory attribute scores per chemical fingerprint).

The method is based on a decomposition of set of chemical fingerprints 254, $\mathbf{X}$, the set of one or more corresponding sensory attribute scores 256, $\mathbf{Y}$, according to:

$$X = ZV^T + E$$

$$Y = ZB + F$$

Where **Z** is an $n \times k$ dimensional matrix of partial least squares (PLS) components, **V** is a $k \times p$ dimensional matrix of PLS loadings, **B** is a vector (or a diagonal matrix) of PLS regression coefficients, and **E** and **F** are residual error terms. The aim of the method 250 is to find a subset of components of **Z** that can be used for a linear regression model to approximate **X** and **Y** as:

$$\hat{X} = ZV^T$$

$$\hat{Y} = ZB$$

The method 250 iteratively finds the values of **Z** based on finding a set of $k < p$ vectors $\{z_k\}$ that are linear combinations of the chemical fingerprint features that maximise the covariance between **X** and **Y**.

The first step of the iteration is to determine 258 the current component of **Z**, $z_i$. To determine a vector, $z_i$, the covariances, $\widetilde{w_i}$, between (the current values of) **X'** and **Y'** are taken, where **X'** and **Y'** are initially **X** and **Y** respectively, e.g.:

$$\widetilde{w_i} = X'^T Y'$$

These can then be normalised to give a set of weight values, $w_i$, e.g.:

$$w_i = \frac{\widetilde{w_i}}{\|\widetilde{w_i}\|}$$

which are used to construct **z;** by taking a weighted sum of corresponding components of **X'**:

$$z_i = w_{1i}X_1 + w_{2i}X_2 \dots w_{pi}X_p$$

Next, a linear regression 260 is performed between $z_i$ and **X'** to determine the corresponding PLS loading, i.e., component of **V**, e.g.:

$$v_i = X'^T z_i / z_i^T z_i$$

and between **z;** and **Y'** to determine the corresponding PLS regression coefficient, i.e., $b_i$, e.g.:

$$b_i = Y'^T z_i / z_i^T z_i$$

The values of **X'** and Y' are then updated 262 using deflation, e.g.:

$$X' = X' - z_i v_i^T$$

$$Y' = Y' - b_i z_i$$

Operations 258 to 262 are repeated k times to generate the partial least squares regression model.

Figure 3 shows a flow diagram of an example method 300 for determining sensory attributes of a processed tobacco product. The method 300 may be performed at least in part by one or more computers operating on one or more locations.

At operation 302, mass spectroscopy data is obtained from a processed tobacco product. The processed tobacco product may be a tobacco blend. Alternatively, the processed tobacco product may be a tobacco heating product sample.

In some examples, the mass spectroscopy data is obtained from a sample from a manufactured batch of the processed tobacco product. In such examples, the method 300 may be used as a quality control method.

At operation 304, one or more chemical fingerprints of the processed tobacco product are determined from the mass spectroscopy data.

At operation 306, the one or more chemical fingerprints of the processed tobacco product are processed using a plurality of machine learning models to obtain a respective score for each of a plurality of sensory attributes for the processed tobacco product.

The plurality of machine learning models may comprise a first plurality of machine learning models. Each machine learning model in the first plurality of machine learning models is configured to take one or more chemical fingerprints of a tobacco blend of the processed tobacco product as input, process the input and output a score for a respective sensory attribute. In other words, each machine learning model in the first plurality of machine learning models is specialised in determining a particular sensory attribute of a tobacco blend.

The plurality of machine learning models may comprise a second plurality of machine learning models. Each machine learning model in the second plurality of machine learning models is configured to take one or more chemical fingerprint of an emission of the processed tobacco product as input, process the input and output a score for a respective sensory attribute.

The plurality of machine learning models may comprise a plurality of partial least square regression models. Alternatively or additionally, the plurality of machine learning models may comprise one or more neural network models, one or more decision tree models, and/or one or more support vector machines. Many other example of machine learning models are possible. In some examples, each machine learning model in the plurality of machine learning models is of the same type, e.g., each machine learning model in the plurality of machine learning models is a partial least square regression model. In other examples, the plurality of machine learning models comprises a mix of machine learning model types.

The scores of the one or more sensory attributes may take values within a predefined range, e.g., score between zero and five, or a score between zero and ten. In some examples, one or more of the scores may take continuous values in the predefined range, e.g., any value between zero and five. In some examples, the one or more of the scores may take discrete values in the predefined range, e.g., integer values in the range zero to ten. In some examples, the plurality of sensory scores are normalised to the same range, i.e., all take values in the same predetermined range. In other examples, the plurality of sensory scores may include scores that lie in different ranges.

The sensory attributes may comprise one or more (e.g., a plurality of) pre-heating sensory attributes for the processed tobacco product, i.e., sensory attributes of the processed tobacco product before it is heated/burned. For example, the pre-heating sensory attributes for the processed tobacco product may comprise one or more of: a rod aroma intensity; and/or an ease of rod insertion.

Alternatively or additionally, the plurality of sensory attributes may comprise a one or more (e.g., a plurality of) first-puff sensory attributes for the processed tobacco product. The first puff attributes relate to initial sensory attributes of the processed tobacco product when heated. The first-puff sensory attributes may comprise one or more of: an impact attribute; an irritation attribute; a visible emission attribute; a taste intensity attribute; a flavour intensity attribute; an emission volume attribute; and/or an emission warmth attribute.

The plurality of sensory attributes may comprise one or more (e.g., a plurality of) overall sensory attributes for the processed tobacco product. The overall sensory attributes for the processed tobacco product relate to sensory attributes of the processed tobacco product during heating. The overall sensory attributes may comprise one or more of: cardboard-paper intensity; a bread-cereal intensity; an emission volume; a tobacco taste intensity; an off notes intensity; an impact attribute; an irritation attribute; a visible emission attribute; a roasted intensity attribute; a spice intensity attribute; a woody intensity attribute; a bitterness attribute; a sweetness attribute; a sourness attribute; a flavour amplitude; an emission warmth; a draw resistance a flavour consistency; and/or an emission consistency.

Alternatively or additionally, the plurality of sensory attributes may comprise a one or more (e.g., a plurality of) aftertaste sensory attributes for the processed tobacco product. The aftertaste attributes relate to sensory experiences subsequent to use of the processed tobacco product. The aftertaste sensory attributes for the processed tobacco product may comprise one or more of: a level of mouth drying/dryness; a level of oily mouth coating; a flavour intensity; a tobacco taste intensity; a hand aroma score; an ease of rod removal; and/or a bitter taste intensity.

In some examples, where the method 300 is being performed as part of a quality control process, the method 300 may further comprise determining that the scores for one or more of the plurality of sensory attributes for the processed tobacco product are not within a respective one or more threshold values of corresponding reference scores for the processed tobacco product.

In response to determining that the scores for one or more of the plurality of sensory attributes for the processed tobacco product are not within the respective one or more threshold values of the corresponding reference scores for the processed tobacco product, the method 300 may further comprise indicating that the manufactured batch of the processed tobacco product is rejected.

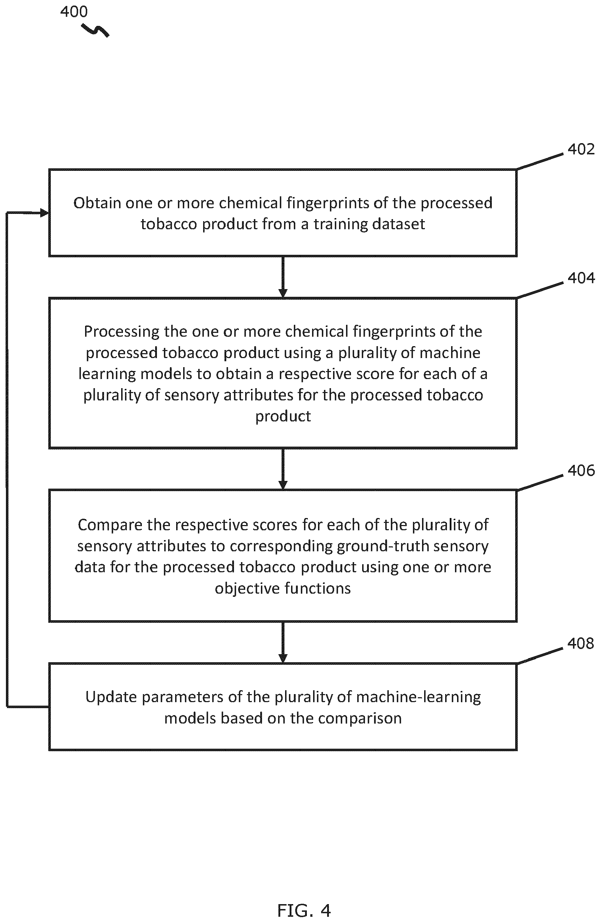

Figure 4 shows a flow diagram of an example method 400 for training a machine-learning model to determine sensory attributes of a processed tobacco product. The method 400 may be performed by one or more computers operating in one or more locations.

At operation 402, one or more chemical fingerprints of a processed tobacco product are received from a training dataset. The training dataset comprises a plurality of training examples, each of which comprises one or more chemical fingerprints of a processed tobacco product and a set of ground truth sensory attribute scores for the processed tobacco product.

The ground truth sensory attribute scores may be derived from human testing of the processed tobacco product. For example, the ground truth sensory attribute scores may be generated using a panel of human testers, each of which assigns respective sensory attribute scores to a processed tobacco product based on their respective experience of the product. A combined score for each sensory attribute may be generated from human tester scores, for example by averaging (e.g., by taking the mean score, median score, or modal score).

The sensory attributes and their corresponding scores may correspond to those described in relation to figure 3.

At operation 404, the one or more chemical fingerprints are processed by one or more machine learning models to generate a respective one or more candidate sensory scores for the processed tobacco product. Each machine learning model processes at least a part of the chemical footprint (e.g., all of the features of the chemical footprint, or a proper subset of the features of the chemical footprint) based on current values of the parameters of the machine learning model (e.g., weights and/or biases of the machine learning model) to generate its respective candidate sensory score.

The machine learning models may be any of the machine learning models described in relation to figure 3.

At operation 406, the score for each of the plurality of sensory attributes is compared to corresponding ground-truth sensory data for the processed tobacco product using one or more objective/loss functions. The loss/objective function may act as a performance metric for the machine learning model. As an example, the one or more objective functions may comprise an L1 or L2 loss. Alternatively or additionally, the one or more objective functions may comprise one or more of a root mean square error of cross validation (RMSECV), a root mean square error of prediction (RMSEP), an R squared ($R^2$) value, and/or an accuracy value.

In some examples, operations 402 to 406 are performed a plurality of times, e.g., a predetermined number of times, before moving on to operation 408.

At operation 408, parameters of the one or more machine learning models are updated based on the comparisons. An optimization process, such as stochastic gradient descent, can be applied to each of the one or more objective functions to determine parameter updates for its respective machine learning model.

Operations 402 to 408 may be repeated a plurality of times, for example until a threshold condition is satisfied. The threshold condition may be a threshold number of iterations, e.g., a threshold number of training epochs or the like. Alternatively or additionally, the threshold condition may be a threshold performance being reached on a test/validation dataset, where the test/validation dataset comprises a plurality of training examples that are not used in the training steps 402-408.

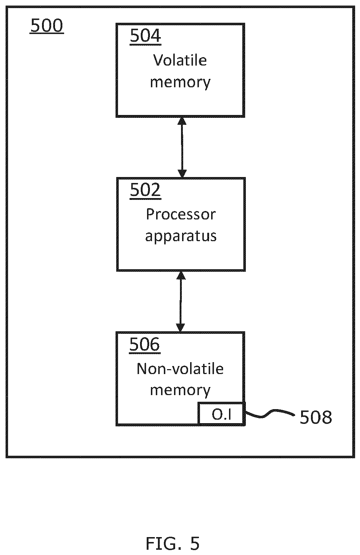

FIG. 5 shows a schematic example of a system/apparatus 500 for performing any of the methods described herein. The system/apparatus shown is an example of a computing device. It will be appreciated by the skilled person that other types of computing devices/systems may alternatively be used to implement the methods described herein, such as a distributed computing system.

The apparatus (or system) 500 comprises one or more processors 502. The one or more processors control operation of other components of the system/-apparatus 500. The one or more processors 502 may, for example, comprise a general-purpose processor. The one or more processors 502 may be a single core device or a multiple core device. The one or more processors 502 may comprise a Central Processing Unit (CPU) or a graphical processing unit (GPU). Alternatively, the one or more processors 502 may comprise specialized processing hardware, for instance a RISC processor or programmable hardware with embedded firmware. Multiple processors may be included.

The system/apparatus comprises a working or volatile memory 504. The one or more processors may access the volatile memory 504 in order to process data and may control the storage of data in memory. The volatile memory 504 may comprise RAM of any type, for example Static RAM (SRAM), Dynamic RAM (DRAM), or it may comprise Flash memory, such as an SD-Card.

The system/apparatus comprises a non-volatile memory 506. The non-volatile memory 506 stores a set of operation instructions 508 for controlling the operation of the processors 502 in the form of computer readable instructions. The non-volatile memory 506 may be a memory of any kind such as a Read Only Memory (ROM), a Flash memory or a magnetic drive memory.

The one or more processors 502 are configured to execute operating instructions 608 to cause the system/apparatus to perform any of the methods described herein. The operating instructions 508 may comprise code (i.e. drivers) relating to the hardware components of the system/apparatus 500, as well as code relating to the basic operation of the system/apparatus 500. Generally speaking, the one or more processors 502 execute one or more instructions of the operating instructions 508, which are stored permanently or semi-permanently in the non-volatile memory 506, using the volatile memory 504 to store temporarily data generated during execution of said operating instructions 508.

Implementations of the methods described herein may be realized as in digital electronic circuitry, integrated circuitry, specially designed ASICs (application specific integrated circuits), computer hardware, firmware, software, and/or combinations thereof. These may include computer program products/non-transitory computer readable media (such as software stored on e.g. magnetic discs, optical disks, memory, Programmable Logic Devices) comprising computer readable instructions that, when executed by data processing apparatus, such as that described in relation to Figure 5, cause the data processing apparatus to perform one or more of the methods described herein.

Any system feature as described herein may also be provided as a method feature, and vice versa. As used herein, means plus function features may be expressed alternatively in terms of their corresponding structure. In particular, method aspects may be applied to system aspects, and vice versa.

Furthermore, any, some and/or all features in one aspect can be applied to any, some and/or all features in any other aspect, in any appropriate combination. It should also be appreciated that particular combinations of the various features described and defined in any aspects of the invention can be implemented and/or supplied and/or used independently.

It will be appreciated that these potential uses listed above for the technology described herein are provided by way of example only, and without limitation. In conclusion, in order to address various issues and advance the art, this disclosure shows by way of illustration various embodiments in which the claimed invention(s) may be practiced. The various embodiments described herein are presented only to assist in understanding and teaching the claimed features. These embodiments are provided as a representative sample of embodiments only, and are not exhaustive and/or exclusive. It is to be understood that advantages, embodiments, examples, functions, features, structures, and/or other aspects of the disclosure are not to be considered limitations on the disclosure as defined by the claims or limitations on equivalents to the claims, and that other embodiments may be utilised, and modifications may be made without departing from the scope of the claims. Various embodiments may suitably comprise, consist of, or consist essentially of, various combinations of the disclosed elements, components, features, parts, steps, means, etc other than those specifically described herein. The disclosure may include other inventions not presently claimed, but which may be claimed in future.

**Claims**

1. A computer implemented method for determining sensory attributes of a processed tobacco product, the method comprising:

   obtaining, by one or more processors, mass spectroscopy data from a processed tobacco product;
   determining, by the one or more processors, one or more chemical fingerprints of the processed tobacco product from the mass spectroscopy data; and
   processing, by the one or more processors, the one or more chemical fingerprints of the processed tobacco product using a plurality of machine learning models to obtain a respective score for each of a plurality of sensory attributes for the processed tobacco product.

2. The method of claim 1, wherein the plurality of machine learning models comprises:

   a first plurality of machine learning models configured to take one or more chemical fingerprints of a tobacco blend of the processed tobacco product as input, and output respective scores for the plurality of sensory attributes; and
   a second plurality of machine learning models configured to take one or more chemical fingerprint of an emission of the processed tobacco product as input, and output respective scores for the plurality of sensory attributes.

3. The method of any of claims 1 or 2, wherein the processed tobacco product comprises a tobacco blend

4. The method of any of claims 1 or 2, wherein the processed tobacco product comprises a tobacco heating product sample.

5. The method of any preceding claim, wherein the plurality of sensory attributes comprises a plurality of pre-heating sensory attributes for the processed tobacco product, optionally wherein the plurality of pre-heating sensory attributes for the processed tobacco product comprises one or more of: a rod aroma intensity; and/or an ease of rod insertion.

6. The method of any preceding claim, wherein the plurality of sensory attributes comprises a plurality of first-puff sensory attributes for the processed tobacco product, wherein the first puff attributes relate to initial sensory attributes of the processed tobacco product when heated, optionally wherein the plurality of first-puff sensory attributes comprises: an impact attribute; an irritation attribute; a visible emission attribute; a taste intensity attribute; a flavour intensity attribute; an emission volume attribute; and/or an emission warmth attribute.

7. The method of any preceding claim, wherein the plurality of sensory attributes comprises a plurality of overall sensory attributes for the processed tobacco product, wherein the plurality of overall sensory attributes for the processed tobacco product relate to sensory attributes of the processed tobacco product during heating, optionally wherein the plurality of overall sensory attributes comprises one or more of: cardboard-paper intensity; a bread-cereal intensity; an emission volume; a tobacco taste intensity; an off notes intensity; an impact attribute; an irritation attribute; a visible emission attribute; a roasted intensity attribute; a spice intensity attribute; a woody intensity attribute; a bitterness attribute; a sweetness attribute; a sourness attribute; a flavour amplitude; an emission warmth; a draw resistance a flavour consistency; and/or an emission consistency.

8. The method of any preceding claim, wherein the plurality of sensory attributes comprises a plurality of aftertaste sensory attributes for the processed tobacco product, wherein the plurality of aftertaste sensory attributes for the processed tobacco product relate to an aftertaste of the tobacco product.

9. The method of claim 8, wherein the plurality of aftertaste sensory attributes for the processed tobacco product comprises one or more of: a flavour intensity; a mouth drying attribute; an oily mouth coating attribute; a tobacco taste intensity; an ease of rod removal attribute; an aroma on hand attribute; and/or a bitter taste intensity.

10. The method of any preceding claim, wherein the plurality of machine learning models comprises a plurality of partial least square regression models.

11. The method of any preceding claim, wherein the mass spectroscopy data is obtained from a sample from a manufactured batch of the processed tobacco product, and wherein the method further comprises:

determining that the scores for one or more of the plurality of sensory attributes for the processed tobacco product are not within a respective one or more threshold values of corresponding reference scores for the processed tobacco product; and

in response to determining that the scores for one or more of the plurality of sensory attributes for the processed tobacco product are not within the respective one or more threshold values of the corresponding reference scores for the processed tobacco product, indicating that the manufactured batch of the processed tobacco product is rejected.

12. The method of any preceding claim, further comprising:

comparing the respective scores for each of the plurality of sensory attributes to corresponding ground-truth sensory data for the processed tobacco product using one or more objective functions; and

updating parameters of the plurality of machine-learning models based on the comparison.

13. The method of claim 12, wherein the ground-truth sensory data comprises signature profiling data obtained from a plurality of human testers.

14. Apparatus comprising one or more processors and a memory, the memory storing computer readable instructions that, when executed by the one or more processors, cause the apparatus to perform a method according to any one or more of the preceding claims.

15. A computer program product storing computer readable instructions that, when executed by a computer, cause the computer to perform a method according to any one or more of claims 1 to 13.

FIG. 1

EP 4 682 532 A1

FIG. 2A

EP 4 682 532 A1

250

252

254    $X$

256    $Y$

258

$$\widetilde{w_i} = \mathbf{X'}^T \mathbf{Y'}$$

$$w_i = \frac{\widetilde{w_i}}{\|\widetilde{w_i}\|}$$

$$z_i = w_i \mathbf{X}_1$$

260

$$\mathbf{v_i} = \mathbf{X'}^T z_i / z_i^T z_i$$

$$b_i = \mathbf{Y'}^T z_i / z_i^T z_i$$

262

$$X' = X' - z_i v_i^T$$

$$Y' = Y' - b_i z_i$$

FIG. 2B

300

302

Obtaining mass spectroscopy data from a processed tobacco product

304

Determine one or more chemical fingerprints of the processed tobacco product from the mass spectroscopy data

306

Processing the one or more chemical fingerprints of the processed tobacco product using a plurality of machine learning models to obtain a respective score for each of a plurality of sensory attributes for the processed tobacco product

FIG. 3

400

402

Obtain one or more chemical fingerprints of the processed tobacco product from a training dataset

404

Processing the one or more chemical fingerprints of the processed tobacco product using a plurality of machine learning models to obtain a respective score for each of a plurality of sensory attributes for the processed tobacco product

406

Compare the respective scores for each of the plurality of sensory attributes to corresponding ground-truth sensory data for the processed tobacco product using one or more objective functions

408

Update parameters of the plurality of machine-learning models based on the comparison

FIG. 4

500

504
Volatile
memory

502
Processor
apparatus

506
Non-volatile
memory

O.I

508

FIG. 5

Europäisches
Patentamt

European
Patent Office

Office européen
des brevets

# EUROPEAN SEARCH REPORT

**Application Number**

EP 24 18 9662

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SOARES FREDERICO L.F. ET AL: "Chemosensory aerosol assessment of key attributes for tobacco products", JOURNAL OF CHEMOMETRICS., vol. 34, no. 12, E3297, 1 December 2020 (2020-12-01), pages 1-15, XP093226522, GB ISSN: 0886-9383, DOI: 10.1002/cem.3297 * abstract * * page 2, paragraph 5 - page 4, paragraph 2 * * page 5, paragraph 3 * * page 9, paragraph 2 * ----- | 1-15 | INV. G01N33/00 G16C60/00 |
| Y | US 2020/256837 A1 (GAO DAQI [CN] ET AL) 13 August 2020 (2020-08-13) * paragraphs [0002], [0003], [0020], [0121], [0154] - [0156] * * figures 1,12,16-18 * ----- | 1-15 | |
| Y | CN 117 934 387 A (NANJING WENCAI INDUSTRIAL INTELLIGENCE RES INSTITUTE CO LTD) 26 April 2024 (2024-04-26) * abstract * * figure 2 * ----- | 11 | TECHNICAL FIELDS SEARCHED (IPC) G01N G16C G06N |

The present search report has been drawn up for all claims

| Place of search | Date of completion of the search | Examiner |
|---|---|---|
| The Hague | 2 December 2024 | Couteau, Olivier |

EPO FORM 1503 03.82 (P4C01)

## ANNEX TO THE EUROPEAN SEARCH REPORT
## ON EUROPEAN PATENT APPLICATION NO.

**EP 24 18 9662**

This annex lists the patent family members relating to the patent documents cited in the above-mentioned European search report.
The members are as contained in the European Patent Office EDP file on
The European Patent Office is in no way liable for these particulars which are merely given for the purpose of information.

**02-12-2024**

| Patent document cited in search report | Publication date | Patent family member(s) | | Publication date |
|---|---|---|---|---|
| US 2020256837 A1 | 13-08-2020 | US | 2020256837 A1 | 13-08-2020 |
| | | WO | 2019085369 A1 | 09-05-2019 |
| CN 117934387 A | 26-04-2024 | NONE | | |

EPO FORM P0459

For more details about this annex : see Official Journal of the European Patent Office, No. 12/82